(19) **Europäisches Patentamt / European Patent Office / Office européen des brevets**

(11) **EP 1 232 725 B1**

(12) **FASCICULE DE BREVET EUROPEEN**

(45) Date de publication et mention de la délivrance du brevet:
**31.10.2007 Bulletin 2007/44**

(51) Int Cl.:
*A61B 6/00* (2006.01)

(21) Numéro de dépôt: **02290368.6**

(22) Date de dépôt: **14.02.2002**

(54) **Procédé de radiographie à double énergie, et dispositif de calibration pour ce procédé**

Röntgenverfahren mittels zweier Röntgenstrahlenergien und Kalibriervorrichtung für dieses Verfahren

Method of dual-energy radiography, and calibration device for this method

(84) Etats contractants désignés:
**DE GB IT**

(30) Priorité: **16.02.2001 FR 0102140**

(43) Date de publication de la demande:
**21.08.2002 Bulletin 2002/34**

(73) Titulaire: **COMMISSARIAT A L'ENERGIE ATOMIQUE**
**75015 Paris (FR)**

(72) Inventeurs:
• **Robert-Coutant, Christine**
  **38410 Saint Martin d'Uriage (FR)**
• **Dinten, Jean-Marc**
  **69008 Lyon (FR)**

(74) Mandataire: **Ilgart, Jean-Christophe**
  **c/o Brevatome,**
  **3, rue du Docteur Lancereaux**
  **75008 Paris (FR)**

(56) Documents cités:
**EP-A- 1 062 912**     **EP-A- 1 072 223**
**US-A- 5 095 499**

**EP 1 232 725 B1**

**Description**

Le sujet de cette invention est un procédé de radiographie à double énergie, ainsi qu'un dispositif de calibration associé à ce procédé.

La radiographie à double énergie consiste à exposer un objet ou un patient à un rayonnement de deux énergies différentes, pour lesquelles les propriétés d'atténuation des matériaux constituant l'objet ou le patient ne varient pas de la même façon. A l'aide d'un modèle de leurs fonctions d'atténuation, on obtient alors des informations sur la densité et la nature des matériaux traversés. En particulier, dans le cas de l'ostéodensitométrie, la masse d'os traversée peut être calculée en la distinguant de la contribution des tissus mous à l'atténuation du rayonnement.

Le fondement théorique de la méthode sera rapidement résumé ci-dessous.

Le flux Ø ayant traversé une longueur 1 d'un matériau de coefficient d'atténuation linéique $\mu$ à partir d'un flux initial de rayonnement $\emptyset_0$ est $\emptyset = \emptyset_0 e^{-\mu l}$. On appelle m la mesure d'atténuation, égale à $\ln\left(\dfrac{I_0}{I}\right)$, où $I_0$ et $I$ sont les signaux mesurés par un même détecteur sous les flux $\emptyset_0$ et $\emptyset$. Dans le cas d'un objet complexe composé d'un grand nombre de matériaux (d'indice i), chacun contribue à l'atténuation d'après sa longueur $L_i$ que les rayons traversent.

Or chaque matériau peut être exprimé, pour la propriété d'atténuation, comme une combinaison linéaire de deux matériaux de base, d'après la formule :

$$\mu = k_1\mu_1 + k_2\mu_2,$$

où $k_1$ et $k_2$ sont des coefficients constants, et $\mu_1$ et $\mu_2$ les atténuations de ces matériaux de base, et on pourra exprimer les longueurs équivalentes $A_1$ et $A_2$ des matériaux de base traversées par le rayonnement par :

$$\begin{cases} A_1 = \sum_i L_i.k_1 \\ A_2 = \sum_i L_i.k_2 \end{cases}$$

Au moyen de ces longueurs équivalentes, les matériaux de base pourront représenter un objet même s'il est en réalité de composition beaucoup plus complexe. -Même si les matériaux composant l'objet sont différents des matériaux de base, la décomposition a un sens. Dans le cas d'examen sur des êtres vivants, les matériaux de base classiques sont le plexiglas (polyméthacrylate) pour simuler les tissus mous et l'hydroxyapatite pour simuler les tissus osseux.

Le système d'équations reliant les mesures aux atténuations $\mu_j$ et aux longueurs équivalentes $A_j$ des matériaux de base est linéaire et donc facile à résoudre si le rayonnement est monochromatique ; mais il ne l'est pas dans les situations réelles, et les longueurs équivalentes sont alors données par des longueurs équivalentes sont alors données par des modèles mathématiques plus compliqués, tels que celui-ci :

$$\begin{cases} A_1 = a_0 + a_1.mBE + a_2.mHE + a_3.mBE.mHE + a_4.m^2_{BE} + a_5.m^2_{HE} \\ A_2 = b_0 + b_1.mBE + b_2.mHE + b_3.mBE.mHE + b_4.m^2_{BE} + b_5.m^2_{HE} \end{cases}$$

qui donne en général une précision suffisante, et où mBE et mHE désignent les mesures d'atténuation à haute et basse énergie, et les « a » et « b » sont des coefficients qu'il faut calculer auparavant par une calibration.

Cette calibration fait appel à un dispositif souvent appelé « fantôme » et qui est composé des matériaux de base évoqués ci-dessus et choisis pour simuler au mieux l'objet de mesure. Ces matériaux sont distribués dans le fantôme de façon à y présenter des régions où les longueurs, traversées par le rayonnement, des matériaux sont distribuées différemment.

Un fantôme classique est construit sous la forme d'un escalier pour chacun des matériaux de mesure, et les escaliers sont superposés de façon que leurs marches soient perpendiculaires. En faisant traverser les escaliers par des rayons verticaux, on obtient ainsi toutes les combinaisons souhaitables entre les diverses épaisseurs des deux matériaux. Le document US-A-5 095 499 décrit un fantôme utilisé en mammographie.

Un autre fantôme est proposé dans le brevet américain 5 493 601 et comprend une série de tubes convergeant vers la source du faisceau et dotés des hauteurs inégalement réparties des deux matériaux. Il a pour but d'offrir des mesures plus exactes que le précédent, notamment grâce à la convergence des tubes vers la source, qui fait parfaitement coïncider la longueur parcourue par les rayons dans les tubes avec la hauteur totale de ceux-ci, et aussi pour réduire le rayonnement diffusé par le contenu des tubes. Les mesures expriment donc directement l'atténuation du rayonnement à travers le fantôme et permettent de calculer sans souci les coefficients recherchés.

Il n'en va pourtant pas de même pour les mesures réalisées à travers l'objet à radiographier, pour lesquelles les conditions favorables exposées ci-dessus ne peuvent être réunies et le rayonnement diffusé ne doit pas être négligé ; il est même particulièrement important pour les sources à faisceau conique, souvent plus que le rayonnement primaire, qui a accompli un trajet rectiligne de la source au détecteur et est seul utile à la mesure. L'estimation puis la correction de ce rayonnement diffusé nécessitent des traitements spécifiques. C'est ainsi qu'il est possible, parmi bien d'autres méthodes, de le mesurer séparément puis de le soustraire du rayonnement total reçu par le détecteur. On dispose pour cela d'un réseau d'éléments absorbants, comme des billes de plomb disposées de façon à former un quadrillage (grille). Les billes sont collées sur du plexiglass et réparties de façon homogène, régulière par exemple selon des lignes et des colonnes de façon à permettre l'interpolation entre les billes, entre l'objet radiographié et le réseau de pixels du détecteur. Les rayons devant passer par ces billes sont interceptés totalement, de sorte qu'il ne parvient aux pixels du détecteur situé sur le chemin de ces rayons que le rayonnement diffusé. Des interpolations numériques permettent ensuite d'estimer, avec une précision suffisante, la répartition du rayonnement diffusé sur l'ensemble des pixels du détecteur. Ce procédé commode présente malheureusement l'inconvénient de devoir soumettre l'objet à une deuxième irradiation, susceptible d'être mal acceptée pour des êtres vivants.

On peut aussi, au contraire, empêcher le rayonnement diffusé de parvenir aux détecteurs en les munissant d'une collimation stricte qui l'intercepte. Les procédés faisant appel à cette technique obligent à recourir à un balayage de la source qui implique un temps d'acquisition long, et le risque que l'objet ne bouge au cours de la mesure.

Des méthodes numériques de genres divers ont encore été proposées pour corriger l'influence du rayonnement diffusé, mais elles ne conviennent généralement bien qu'à des objets particuliers. On doit conclure que le brevet cité plus haut ne semble pas proposer un progrès sensible, puisque le rayonnement diffusé n'est écarté des mesures que pour l'étape de calibration.

L'objet de l'invention est de corriger l'influence du rayonnement diffusé d'une autre manière, en le laissant apparaître à la calibration, mais de façon analogue à ce qu'il fait à la radiographie de l'objet, ce qui permet de le corriger par un même procédé numérique, sans qu'on ait à craindre des discordances dans la qualité de ce procédé.

Pour résumer, l'invention concerne avant tout un dispositif de calibration d'un système de radiographie à double énergie, comprenant un jeu de blocs présentant différentes épaisseurs d'un premier matériau, caractérisé en ce que les blocs sont munis d'évidements et en ce que le dispositif comprend encore des inserts à combler les évidements et comprenant différentes répartitions de hauteurs, les hauteurs et les épaisseurs étant considérées dans une direction identique, entre le premier matériau et un second matériau ; ainsi qu'un procédé de radiographie à faisceau conique à double énergie, comprenant une estimation d'épaisseurs de matériaux d'un objet radiographique par une combinaison numérique de mesures d'atténuation des énergies, impliquant une calibration de coefficients de la combinaison, caractérisé en ce que la calibration est mesurée par une radiographie d'un dispositif de calibration conforme à l'une quelconque des revendications précédentes, et qu'un rayonnement diffusé affectant la radiographie du dispositif de calibration est estimé tout en donnant un critère d'estimation utilisé ensuite pour estimer un rayonnement diffusé affectant la radiographie de l'objet.

L'invention sera maintenant décrite au moyen des figures :

- la figure 1 représente un organigramme de l'invention ; et
- les figures 2 et 3 représentent une vue de côté et une vue de dessus d'un fantôme de calibration utilisé pour satisfaire aux exigences exposées plus haut.

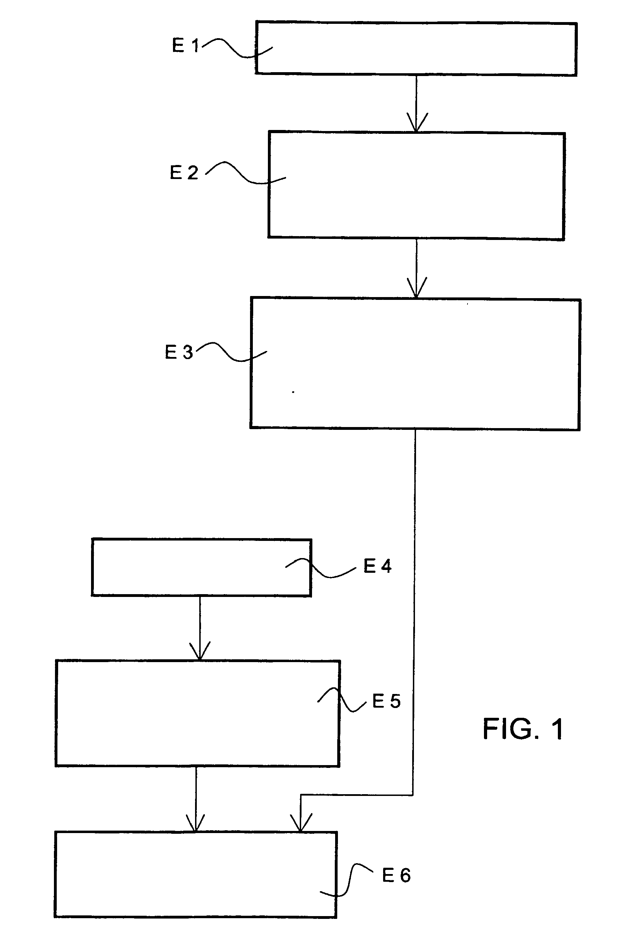

On se reporte à la figure 1.

A l'étape E1, une acquisition de calibration est faite par une radiographie à travers le fantôme décrit plus loin, ce qui donne des mesures brutes à haute et basse énergie. A l'étape E2, un traitement préliminaire d'estimation et de soustraction du rayonnement diffusé de ces mesures est fait pour calculer les mesures corrigées mHe et mBe des formules précédentes. Ensuite, l'étape E3 consiste en une estimation des paramètres du modèle, notamment les coefficients des polynômes a et b à partir des mesures et des épaisseurs connues que le rayonnement traverse dans le fantôme. D'autre part, une acquisition, à travers l'objet de mesure, est faite à l'étape E4, puis un traitement préliminaire est fait à l'étape E5 ; il est semblable à celui de l'étape E2 et son but est également d'estimer, puis de soustraire le rayonnement diffusé qui affecte les mesures. L'étape finale E6 consiste à exploiter les mesures corrigées effectuées à travers l'objet et d'appliquer le modèle, et avant tout les coefficients a et b calculés à l'étape E3, afin de déduire les longueurs équivalentes des matériaux de base a1 et a2 dans l'objet.

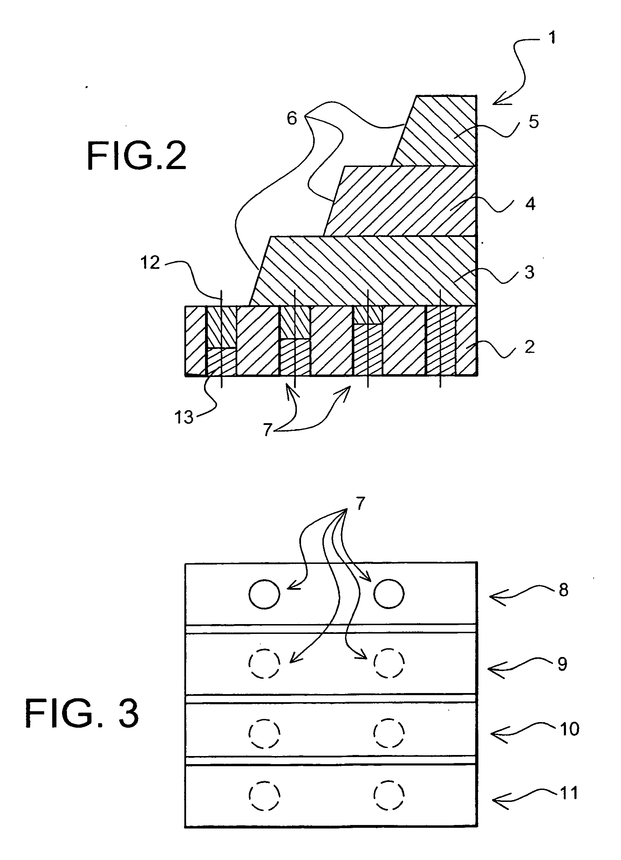

Un exemple de fantôme utilisé est représenté aux figures 2 et 3.

Il comprend quelques blocs d'épaisseurs différentes d'un des matériaux de base, notamment le plexiglas. On pourrait utiliser un jeu de blocs séparés, à travers lesquels les mesures seraient faites successivement, mais on peut aussi unir les blocs en formant un solide à profil d'escalier 1 comprenant différentes couches, ici au nombre de quatre, et numérotées de 2 à 5 de bas en haut, les couches finissant, en général, par des faces dressées 6 ayant une orientation en dépouille, afin de réduire les composantes à haute fréquence du rayonnement diffusé. De plus, des rangées d'inserts sont prévues sous les différents blocs, en comblant des évidements opérés ici à travers la couche inférieure 2. On rencontre, plus précisément, quatre rangées de deux inserts 7 chacune, numérotées de 8 à 11 et qui s'étendent respectivement sous la surface supérieure de la couche inférieure 2, sous la deuxième couche 3, sous les couches 3 et 4 et sous les trois couches supérieures 3 à 5. Les inserts 7 comprennent, en général, une portion en hydroxyapatite 12 et une portion en plexiglas 13. Les portions 12 et 13 ont une même hauteur totale, mais des hauteurs respectives différentes dans chaque rangée 8 à 11, de sorte que les rayons originaires de la source 14 et qui passent par chacun des inserts 7, traversent des combinaisons différentes d'épaisseurs des deux matériaux. De plus, il est produit un rayonnement diffusé analogue à celui qui est produit dans un être vivant. On est redevable de cette similitude à la constitution même du fantôme 1, puisque les matériaux qui le composent, outre qu'ils simulent bien les tissus mous et les os, ont des proportions et des répartitions analogues. En particulier, les inserts 7 sont suffisamment éloignés pour ne pas recevoir de rayonnement diffusé originaire des inserts voisins, mais seulement de la matière de base des couches 2 à 5.

On décrira, pour la fin de cet exposé, un procédé d'estimation du rayonnement diffusé, utilisable pour les étapes E2 et E5 ; ce procédé fait cependant partie d'une autre invention et n'est donc donné que par souci de compléter la description de celle-ci et de prouver l'intérêt du fantôme 1.

A l'étape E2, un rayonnement semblable à celui que subira l'objet et de flux $\emptyset_0$ est projeté sur le fantôme 1, et un rayonnement total $\emptyset_t$, somme d'un rayonnement primaire $\emptyset$ et du rayonnement diffusé $\emptyset_d$, est capté par des détecteurs placés derrière le fantôme 1 et mesurant donc l'atténuation du rayonnement initial $\emptyset_0$ à travers lui. En particulier, on obtient des mesures de rayonnement ayant passé par chacun des inserts 7, aussi bien pour la haute que pour la basse énergie.

Les mêmes mesures sont répétées après avoir placé des absorbeurs, telles des billes de plomb, entre la source et le fantôme de façon à intercepter le rayonnement primaire et à ne plus mesurer que le rayonnement diffusé par les détecteurs derrière les billes de plomb. On en déduit par interpolation la valeur du rayonnement diffusé $\emptyset_d$ en fonction des hauteurs traversées des deux matériaux de base pour chacune des énergies.

L'acquisition des mesures à travers l'objet donne aussi deux jeux de valeurs de rayonnement total $\emptyset_t$ à haute et basse énergie. Le rayonnement $\emptyset_d$ à travers l'objet peut être estimé d'après les mesures derrière les billes de plomb à travers le fantôme 1 et la relation entre $\emptyset_t$, $\emptyset_d$ et $\emptyset$ obtenue à travers lui pour les deux énergies, en supposant que la même relation s'applique à travers l'objet.

Quoique l'invention trouve utilité pour les faisceaux coniques, son emploi n'est pas limité à eux ; on peut concevoir que les étapes d'estimation et de correction du rayonnement diffusé pourraient être omises dans des situations où il serait moins important, puisqu'il affecterait les mesures à travers le fantôme 1 et l'objet pareillement, grâce à leur similitude de structure, et que son influence disparaîtrait au calcul des longueurs équivalentes $A_1$ et $A_2$.

## Revendications

1. Dispositif (1) de calibration d'un système de radiographie à faisceau conique à double énergie, comprenant un jeu de blocs présentant différentes épaisseurs d'un premier matériau (13), **caractérisé en ce que** les blocs sont munis d'évidements et **en ce que** le dispositif comprend encore des inserts (7) permettant de combler les évidements lesdits inserts, comprenant différentes répartitions de hauteurs entre un premier matériau (13) et un second matériau (12) les hauteurs des inserts et les épaisseurs des blocs étant dans une direction identique.

2. Dispositif de calibration suivant la revendication 1, **caractérisé en ce que** les blocs sont assemblés en une forme d'escalier et les inserts sont répartis en rangées (8 à 11) dans la couche inférieure (2) de l'escalier, les rangées étant situées sous des blocs différents.

3. Dispositif de calibration suivant la revendication 2, **caractérisé en ce que** l'escalier présente des faces dressées (6) en dépouille.

**4.** Procédé de radiographie à faisceau conique à double énergie, comprenant une estimation d'épaisseurs de matériaux d'un objet radiographique par une combinaison numérique de mesures d'atténuation des énergies, impliquant une calibration de coefficients (a, b) de la combinaison, **caractérisé en ce que** la calibration est mesurée par une radiographie d'un dispositif de calibration conforme à l'une quelconque des revendications précédentes, et qu'un rayonnement diffusé affectant la radiographie du dispositif de calibration est estimé tout en donnant un critère d'estimation utilisé ensuite pour estimer un rayonnement diffusé affectant la radiographie de l'objet.

## Claims

**1.** Device (1) for calibrating a system for double energy conical beam radiography, comprising an assembly of blocks of different thicknesses (2 to 5) of a first material (13), **characterized in that** the blocks are provided with recesses and **in that** the device further comprises inserts (7) making it possible to fill the recesses, said inserts comprising different height distribution between a first material (13) and a second material (12), the heights of the inserts and the thicknesses of the blocks being considered in an identical direction.

**2.** Calibration device according to claim 1, **characterized in that** the blocks are assembled in stepped form and the inserts are divided into rows (8 to 11) in the lower layer (2) of the steps, the rows being located under different blocks.

**3.** Calibration device according to claim 2, **characterized in that** the steps have tapered faces (6).

**4.** Method for radiography with a double energy conical beam, comprising an estimation of thicknesses of materials of a radiographic subject by a digital combination of measurements of energy attenuation, involving calibration of coefficients (a, b) of the combination, **characterized in that** the calibration is measured by a radiography of a calibration device according to any one of the preceding claims, and **in that** scattered radiation affecting the radiography of the calibration device is estimated while providing an estimation criterion used afterwards to estimate scattered radiation affecting the radiography of the subject.

## Patentansprüche

**1.** Kalibriervorrichtung (1) eines Röntgensystems mittels konischem Zweienergien-Röntgenstrahl, einen Satz Blöcke unterschiedlicher Dicke aus einem ersten Material (13) umfassend, **dadurch gekennzeichnet, dass** die Blöcke mit Aussparungen versehen sind, und **dadurch**, dass die Vorrichtung außerdem Inserts (7) umfasst, welche die Aussparungen füllen, wobei die genannten Inserts unterschiedliche Höhenverteilungen zwischen einem ersten Material (13) und einem zweiten Material (12) aufweisen und dabei die Höhen der Inserts und die Dicken der Blöcke in einer Richtung identisch sind.

**2.** Kalibriervorrichtung nach Anspruch 1, **dadurch gekennzeichnet, dass** die Blöcke nach Art einer Treppe vereinigt sind und die Inserts in Form von Reihen (8 bis 11) in der untersten Schicht (2) der Treppe verteilt sind, wobei die Reihen sich unter verschiedenen Blöcken befinden.

**3.** Kalibriervorrichtung nach Anspruch 2, **dadurch gekennzeichnet, dass** die Treppe schräge Stirnflächen (6) aufweist.

**4.** Röntgenverfahren mittels konischem Zweienergien-Röntgenstrahl, eine Schätzung der Materialdicke eines Röntgenobjekts durch eine numerische Kombination von Dämpfungsmessungen der Energien umfassend, die eine Kalibrierung von Koeffizienten (a, b) der Kombination mit einschließt, **dadurch gekennzeichnet, dass** die Kalibrierung gemessen wird durch eine Röntgenaufnahme einer Kalibriervorrichtung nach einem der vorangehenden Ansprüche, und dass eine die Röntgenaufnahme der Kalibriervorrichtung beeinflussende Streustrahlung geschätzt wird und dabei ein Schätzkriterium liefert, das anschließend benutzt wird, um eine die Röntgenaufnahme des Objekts beeinflussende Streustrahlung zu schätzen.

FIG. 1

FIG.2

FIG. 3

**EP 1 232 725 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5095499 A